# EUROPÄISCHE PATENTANMELDUNG

(11) **EP 2 506 213 A2**
(43) Veröffentlichungstag der Anmeldung: **03.10.2012**
(21) Anmeldenummer: 12004864.0
(22) Anmeldetag: 28.10.2009
(51) Int. Cl.: G06T 7/00, A61C 13/00, A61B 5/00, A61B 5/107, A61C 7/00, A61B 5/11, A61C 19/045

(54) **Gebissbild-Simulationsvorrichtung**

(30) Priorität: 28.10.2008 DE 202008014344 U
(62) Teilanmeldung aus: 09013578.1
(71) Anmelder: Edinger-Strobl, Verena, 6020 Innsbruck (AT)
(72) Erfinder: Edinger-Strobl, Verena, 6020 Innsbruck (AT)
(74) Vertreter: Thoma, Michael

(57) **Zusammenfassung**

Die vorliegende Erfindung betrifft eine Gebissbild-Simulationsvorrichtung zur Simulation eines Gebissbilds zur Behandlungsergebnis-Visualisierung und/oder Generierung von Daten für die Herstellung von Zahnersatzteilen, mit einer Ist-Bilderzeugungsvorrichtung zur Erzeugung einer Ist-Darstellung des Gebisses in seinem Ist-Zustand, einer Bild-Manipulationsvorrichtung zur Manipulation von Bilddaten der Ist-Darstellung anhand einer vorgebbaren Gebiss- und/oder Zahnbehandlungsfunktion, und einer Soll-Bilderzeugungsvorrichtung zur Erzeugung einer Soll-Darstellung des Gebisses in seinem Soll-Zustand anhand der Ist-Darstellung und der manipulierten Bilddaten. Erfindungsgemäß umfasst die Bildmanipulationsvorrichtung der Gebissbildsimulationsvorrichtung eine Segmentierungsvorrichtung zur automatischen Segmentierung eines Zahns und/oder eines Gebissteils in der Ist-Darstellung in Abhängigkeit der vorgegebenen Zahnbehandlungsfunktion sowie einen Segmentmanipulator zur automatischen Manipulation des segmentierten Bildausschnitts in Abhängigkeit der vorgegebenen Zahnbehandlungsfunktion.

## Beschreibung

Die vorliegende Erfindung betrifft eine Gebissbild-Simulationsvorrichtung zur Simulation eines Gebissbilds zur Behandlungsergebnis-Visualisierung und/oder Generierung von Daten für die Herstellung von Zahnersatzteilen, mit einer Ist-Bilderzeugungsvorrichtung zur Erzeugung einer Ist-Darstellung des Gebisses in seinem Ist-Zustand, einer Bild-Manipulationsvorrichtung zur Manipulation von Bilddaten der Ist-Darstellung anhand einer vorgebbaren Gebiss- und/oder Zahnbehandlungsfunktion, und einer Soll-Bilderzeugungsvorrichtung zur Erzeugung einer Soll-Darstellung des Gebisses in seinem Soll-Zustand anhand der Ist-Darstellung und der manipulierten Bilddaten.

Bei Zahnbehandlungen und der Herstellung von Zahnersatz ist es bislang trotz aller bekannter zahnärztlicher Hilfsmittel schwierig, vorab den Zahnersatz bzw. die Zahnveränderung, beispielsweise durch Abschleifen, Bleichen, Überkronen und dergleichen, genau passend zu konfigurieren, so dass ein stimmiges Gebissbild entsteht, in dem ein beispielsweise einzusetzender Ersatzzahn sich passgenau in die vorhandene Zahnlücke einfügt und optisch mit den restlichen Zähnen harmoniert und nicht zuletzt auch dem Patienten gefällt. Hierzu ist es bekannt, Behandlungsergebnisse mittels Vorher-/Nachher-Bilder anderer Patienten, die eine ähnliche Behandlung hatten, zu visualisieren. Weiters gibt es Schaumodelle, die verschiedene Behandlungsalternativen vorzeigbar machen. Es gibt weiters Computerprogramme zur Demonstration verschiedener Behandlungen an virtuellen Modellen oder mit Bildsequenzen oder Videos von Fällen anderer Patienten.

Dies ist jedoch nur bedingt hilfreich, da es nicht individuell an die Gegebenheiten des jeweiligen Patienten angepasst ist. Durch Anfertigung von Provisorien lässt sich in manchen Fällen das Behandlungsziel vorab visualisieren, dies führt jedoch zu zusätzlichen Kosten und ist zeitraubend. In manchen Fällen ist das Ergebnis der Behandlung überhaupt nicht oder nur sehr schwer für den Patienten vorstellbar und vor der Behandlung am Patienten nicht visualierbar, so z. B. bei Zahnaufhellungen, Füllungstherapien, Kieferorthopädischen Behandlungen, Kieferchirurgischen Behandlungen. Es wäre deshalb wünschenswert, das Ergebnis einer jeweiligen Zahnbehandlungsfunktion möglichst detailgetreu vor Behandlungsbeginn simulieren und visualisieren zu können, so dass der Zahnarzt gemeinsam mit dem Patienten die optimale Konfiguration des beispielsweise einzusetzenden Ersatzzahnes festlegen kann.

Hiervon abgesehen ist die Auswahl der passenden Zahnbehandlung wie z.B. des passenden Zahnersatzes für den Zahnarzt bzw. den Zahntechniker eine zunehmend mühsame Tätigkeit, da der zur Verfügung stehende Auswahlpool hierfür zunehmend größer wird. Es gibt z. B. Ersatzzahnrohlinge bzw. -materialien in unterschiedlichsten Formvarianten und Farbtonnuancen, die an sich alle durchgesehen werden müssten, um den optimal passenden Zahnersatz auszusuchen bzw. den nächstkommenden Zahnersatzrohling bzw. das nächstkommende Material zu wählen, der bzw. das den Umfang der notwendigen Nacharbeit minimiert und ein bestmögliches Ergebnis ermöglicht.

Weiterhin wurde bereits versucht, für die CAD-CAM Fertigung von Zahnersatz die Erfassung der Umrisskonturen des zu ersetzenden Zahnes bzw. die Bestimmung der Konturdaten des zu fertigenden Zahnes zu automatisieren. Hierzu werden in der Regel komplizierte dreidimensionale Erfassungsverfahren eingesetzt wie beispielsweise Laserscannen oder Lichtebenenschnittverfahren, die dreidimensionale Konturdaten generieren, anhand derer automatische Fertigungswerkzeuge wie CAM-Fräser gesteuert werden. Derartige dreidimensionale Zahnerfassungsvorrichtungen verarbeiten jedoch eine Unmenge an Daten und führen eine Vielzahl von Berechnungsschritten aus, was eine äußerst leistungsfähige Hardware erfordert, die in Arztpraxen regelmäßig nicht zur Verfügung steht.

Hiervon ausgehend liegt der vorliegenden Erfindung die Aufgabe zugrunde, eine verbesserte Gebissbild-Simulationsvorrichtung zu schaffen, die Nachteile des Standes der Technik vermeidet und Letzteren in vorteilhafter Weise weiterbildet. Vorzugsweise soll mit einfachen Mitteln bei geringen zu verarbeitenden Datenmengen eine möglichst detailgetreue Gebissbild-Simulation und -Visualisierung erzielt werden, die das Ergebnis einer Zahnbehandlung eines bestimmten Patienten im voraus, d.h. schon vor Durchführung der Zahnbehandlung wiedergibt, um dem Patienten und Arzt eine Entscheidungshilfe zu geben.

Erfindungsgemäß wird diese Aufgabe durch eine Gebissbild-Simulationsvorrichtung gemäß Anspruch 1 gelöst. Bevorzugte Ausgestaltungen der Erfindung sind Gegenstand der abhängigen Ansprüche.

Es wird also vorgeschlagen, in der erzeugten Ist-Darstellung des Gebisses den zu behandelnden Zahn bzw. die zu behandelnden Zähne und/oder den zu behandelnden Gebissteil mittels einer intelligenten Segmentierungsvorrichtung automatisch in Abhängigkeit der vorgegebenen Zahnbehandlungsfunktion und ggf. weiterer individueller Patientenmerkmale wie bspw. Geschlecht, Alter, Kieferform, Lachlinie und dgl. zu segmentieren und den entsprechenden Bildabschnitt in Form von Pixeln zu identifizieren bzw. auszuschneiden, so dass sozusagen der die der Zahnbehandlung unterliegenden Zähne zeigende Bildausschnitt isoliert wird. Je nachdem, welche Zahnbehandlungsfunktion vorzunehmen ist, wird dann dieser isolierte Bildausschnitt manipuliert, indem die Pixeldarstellung in Abhängigkeit der vorzunehmenden Zahnbehandlung transformiert wird, während die restliche Ist-Darstellung des Gebisses unverändert bleibt, wodurch detailliert und realitätsnah die Auswirkung der vorzunehmenden Zahnbehandlung visualisiert werden kann. Als Ist- und Soll-Darstellungen werden dabei insbesondere zweidimensionale Bilder bzw. Darstellungen verwendet, die zur Visualisierung des Ergebnisses der geplanten Behandlung auf einem Bildschirm ausreichenden sind und die verarbeitete Datenmenge signifikant reduzieren. Erfindungsgemäß umfasst die Bildmanipulationsvorrichtung der Gebissbildsimulationsvorrichtung eine Segmentierungsvorrichtung zur automatischen Segmentierung eines Zahns und/oder eines Gebissteils in der Ist-Darstellung in Abhängigkeit der vorgegebenen Zahnbehandlungsfunktion sowie einen Segmentmanipulator zur automatischen Manipulation des segmentierten Bildausschnitts in Abhängigkeit der vorgegebenen Zahnbehandlungsfunktion. Die vorzunehmende Zahnbehandlungsfunktion kann dabei grundsätzlich unterschiedlicher Natur sein. Insbesondere kann die Zahnbehandlungsfunktion das Einsetzen eines Ersatzzahnes, mehrerer Ersatzzähne oder eines ganzen Gebissteiles sein, wobei als Gebissteil auch ein ganzes Ersatzgebiss gemeint sein kann. Alternativ oder zusätzlich kann die Gebissbild-Simulationsvorrichtung auch dazu benutzt werden, eine Veränderung der Zahnstellung zu simulieren und visualisieren, beispielsweise durch eine Zahnspangenbehandlung, mittels derer ein einzelner Zahn oder mehrere Zähne in eine andere Ausrichtung gezwungen werden. Dies kann beispielsweise auch dazu benutzt werden, zu simulieren, ob ein bislang schief stehender, herunterzuziehender Zahn in eine vorhandene Zahnlücke überhaupt hineinpasst oder ob ggf. weitere Maßnahmen vorgenommen werden müssen. Andererseits kann mit der Gebissbild-Simulationsvorrichtung auch ein Bleaching einzelner oder mehrerer Zähne simuliert werden. Alternativ oder zusätzlich können auch Maßnahmen und Veränderungen an dem Gebiss zugehörigen Geweben wie Kieferknochen oder Weichteile wie Lippen und Wangen simuliert werden.

Je nachdem, welche Zahnbehandlungsfunktion auszuführen ist, segmentiert die Segmentierungsvorrichtung die entsprechenden Zähne bzw. Gebiss- oder Zahnbereiche durch Identifikation der diese wiedergebenden Bildpixel, so dass der Segmentmanipulator die entsprechenden Bildpixel in Abhängigkeit der vorzunehmenden Behandlungsfunktion manipulieren kann. Der Segmentmanipulation kann hierbei die erhältliche Ist-Darstellung des Gebisses hinsichtlich Größe, Form, Farbe, Sättigung, Ausrichtung und/oder Position manipulieren.

Die vorzunehmende Zahnbehandlungsfunktion kann in vorteilhafter Weise manuell vorgegeben werden, beispielsweise durch Anklicken eines Funktionssymbols in einer Zahnbehandlungsfunktions-Menüleiste einer Steuerungsbildschirmdarstellung. Alternativ oder zusätzlich kann auch eine automatische Auswahl der vorzunehmenden Zahnbehandlungsfunktion in Abhängigkeit der Ist-Darstellung erfolgen. Enthält beispielsweise die Ist-Darstellung des Gebissbilds einen Zahn mit einer schräg abgeschlagenen Zahnkante, kann dies von einem Gebissbildanalysator automatisch erkannt und die entsprechende Zahnbehandlungsfunktion - im genannten Fall vorteilhafterweise das Einsetzen eines Zahnersatzes - vorgegeben werden.

Die Segmentierung eines zu behandelnden Zahnes bzw. Gebissteiles in der Ist-Darstellung desselben kann grundsätzlich auf verschiedene Art und Weise erfolgen. In vorteilhafter Weiterbildung der Erfindung kann die Segmentierung durch ein Bereichswachstumsverfahren erfolgen, wobei vorteilhafterweise ein Saatpunkt in dem zu segmentierenden Zahn bzw. Gebissabschnitt, oder auch außerhalb des zu segmentierenden Zahns bzw. Gebissabschnitts gesetzt wird. Von diesem Saatpunkt ausgehend werden benachbarte Bildpixel anhand zumindest eines Homogenitätskriteriums dahingehend beurteilt, ob sie noch zum selben Bildbereich oder nicht gehören.

Alternativ oder zusätzlich kann die Segmentierungsvorrichtung vorteilhafterweise auch mittels eines sog. Active Snake-Verfahrens arbeiten, bei dem eine aktive Konturbestimmungseinrichtung zur Bestimmung der Kontur eines zu segmentierenden Zahn- und/oder Gebissteils eine parametrisierte Kurve - die sogenannte snake - zunächst vorgibt und dann in Abhängigkeit gewisser Eigenschaften von Bildpunkten, die innerhalb und/oder außerhalb der Kurve liegen, die Form der Kurve verändert, sozusagen ausbeult und einbeult, zieht und streckt bzw. staucht und dergleichen. Insbesondere kann dabei als Kurve eine Umrisskurve vorgegeben werden, die näherungsweise bereits einer üblichen Zahnkontur entspricht, bspw. anhand der Durchschnittswerte abgespeicherte Zahnumrisslinien, wobei die vorgebbare Umrisskurve anhand verschiedener Kriterien wie beispielsweise die Lage des zu segmentierenden Zahns im Gebiss auswählbar ist, um bereits ausgangsmäßig eine größtmögliche Übereinstimmung zu haben. Die zunächst vorgegebene Schlange bzw. Kurve wird sodann vorteilhafterweise durch eine Gradientenvektor-Bestimmungseinrichtung zur Bestimmung von Gradientenvektoren anhand von Bildpunkteigenschaften eines von der parametrisierten Kurve umschlossenen Bildbereichs und/oder eines die parametrisierte Kurve umgebenden Bildbereichs verformt und somit in Abhängigkeit erfasster Eigenschaften des erzeugten Ist-Bildes der zu bearbeitenden Gebisses verformt. Hierzu kann eine in Abhängigkeit der bestimmten Gradientenvektoren arbeitende Konturformungseinrichtung zur Verformung der parametrisierten Kurve in Abhängigkeit der bestimmten Gradientenvektoren vorgesehen sein.

Alternativ oder zusätzlich kann die Segmentierung mittels eines zellulären Automaten erfolgen. Zelluläre oder auch zellulare Automaten dienen der Modellierung räumlich diskreter dynamischer Systeme, wobei die Entwicklung einzelner Zellen zum Zeitpunkt t + 1 primär von den Zellzuständen in einer vorgegebenen Nachbarschaft und vom eigenen Zustand zum Zeitpunkt t abhängt. Ein solcher Zellularautomat ist dabei insbesondere durch folgende Größen festgelegt:
- ein Raum R (Zellraum)
- eine endliche Nachbarschaft N
- eine Zustandsmenge Q
- eine lokale Überführungsfunktion δ: Q^{N} → Q

Der Zellraum besitzt hierbei eine gewisse Dimensionalität, er ist in der Regel 1- oder 2-dimensional, kann aber durchaus auch höherdimensional sein. Der Zellularautomat nimmt dabei durch seine globale Konfiguration eine Abbildung aus dem Zellraum in die Zustandsmenge vor, das heißt man ordnet jeder Zelle des Automaten einen Zustand zu. Der Übergang einer Zelle von einem Zustand (lokale Konfiguration) in den nächsten wird durch Zustandsübergangsregeln definiert, die deterministisch oder stochastisch sein können. Die Zustandsübergänge erfolgen für alle Zellen nach derselben Überführungsfunktion und gleichzeitig. Die Zellzustände können wie die Zeitschritte diskret sein. Nur wenige Zustandswerte reichen zur Simulation selbst hochkomplexer Systeme aus. Durch Bestimmung der genannten endlichen Nachbarschaft N kann der jeweils interessierende Bereich segmentiert werden. Auch bei der Segmentierung mittels eines zellulären Automaten kann von einem oder mehreren Saatpunkten ausgegangen werde, die innerhalb und/oder außerhalb des zu segmentierenden Bildabschnitts liegen können.

Als Homogenitätskriterium für die Segmentierung können hierbei verschiedene Bildpunkteigenschaften herangezogen werden. Insbesondere kann bei der Segmentierung von Zähnen vorteilhafterweise der Grauwert eines jeweiligen Bildpunktes analysiert werden, da sich die regelmäßig weißen Zähne von Nachbarkonturen gerade im Grauwert stark unterscheiden werden.

Alternativ oder zusätzlich kann als Homogenitätskriterium auch ein Farbton, eine Sättigung und/oder eine Helligkeit des jeweiligen Bildpunktes analysiert werden.

Vorteilhafterweise können mehrere Eigenschaften eines Bildpunktes als Homogenitätskriterium herangezogen werden. Nach einer vorteilhaften Weiterbildung der Erfindung kann das Bereichswachstumsverfahren durch den sog. RGB-Farbraum umfassend die Primärfarben Rot, Grün und Blau, genauer gesagt mittels eines Farbvektors, der die Primärfarben Rot, Grün und Blau eines Bildpunktes angibt, gesteuert werden. Alternativ oder zusätzlich kann auch der HSB-Farbraum bestehend aus dem Farbton, der Sättigung und der Helligkeit herangezogen werden. In diesem Fall wird ein den Farbton, die Sättigung und die Helligkeit angebender Farbvektor eines jeden Bildpunkts als Homogenitätskriterium verwendet.

Vorteilhafterweise ist hierbei in Weiterbildung der Erfindung eines Vergleichseinrichtung vorgesehen, mittels derer der Wert der untersuchten Eigenschaft eines jeweiligen Bildpunkts, insbesondere die Richtung und die Länge des zuvor genannten Farbvektors mit einem Referenzwert dieser Eigenschaft, insbesondere einem Referenzvektor verglichen wird und die sich ergebende Differenz des Vergleichs mit einem Schwellwert verglichen wird. Überschreitet die Abweichung ein zulässiges Maß, wird der Bildpunkt als nicht mehr zum selben Bildbereich zugehörig identifiziert, während umgekehrt bei nur kleinen, d.h. unter dem Schwellwert liegenden Abweichungen die umgekehrte Zuordnung getroffen wird.

Der für den genannten Vergleich herangezogene Vergleichswert, also insbesondere der Vergleichsvektor des jeweiligen Farbraums und/oder der entsprechende Grauwert kann grundsätzlich in verschiedener Art und Weise festgelegt werden. Nach einer besonders einfachen Ausbildung der Erfindung kann der Referenzwert des Homogenitätskriteriums der entsprechende Eigenschaftswert des Saatpunkts sein. Dem liegt die Überlegung zugrunde, dass der Saatpunkt regelmäßig in einem zentralen Bereich des jeweiligen Bildabschnitts gesetzt ist, der sicher dem jeweiligen Bereich zugehörig ist.

Um jedoch auch bei graduellen Veränderungen die Bereichsgrenze deutlich zu identifizieren, beispielsweise bei sich kontinuierlich verändernden Zahnverfärbungen die Zahngrenze sicher zu identifizieren, kann in alternativer Weiterbildung der Erfindung vorgesehen sein, dass der Referenzwert für das Homogenitätskriterium adaptiv festgelegt wird, beispielsweise dergestalt, dass der entsprechende Eigenschaftswert eines zuvor untersuchten und benachbarten Bildpixels herangezogen wird. Vorteilhafterweise bestimmt ein adaptiver Vergleichswertgenerator jedoch einen Mittelwert mehrerer Eigenschaftswerte mehrerer zuvor untersuchter Bildpixel. Dies können die unmittelbar benachbarten Bildpixel sein, wobei als zusätzliches Korrekturglied jedoch auch ein Mittelwert des entsprechenden Eigenschaftswerts aller zuvor untersuchten Bildpixel des Bereichs herangezogen wird. Hierdurch können einerseits graduelle Eigenschaftsänderungen wie Zahnverfärbungen berücksichtigt, andererseits jedoch Überlagerungen in Randbereichen nicht zu weit toleriert werden.

In Weiterbildung der Erfindung kann der Bildanalysator einen Gewichtungsgeber zur Gewichtung einzelner Bildpunkte anhand ihrer Abweichung von einem zuvor bestimmten Mittelwert und/oder anhand ihres Abstands von dem Saatpunkt aufweisen. Dem liegt die Überlegung zugrunde, dass einzelne Bildpunkte bei vorliegend bestimmter Eigenschaften sicherer als andere Bildpunkte zu dem gesuchten Bereich gehören und/oder für die Bestimmung des gesuchten Bereichs wichtiger sind. Wird beispielsweise festgestellt, dass ein Bildpunkt hinsichtlich des ihn identifizierenden Farbraumvektors identisch mit dem zuvor bestimmten Mittelwert ist, kann mit relativ hoher Sicherheit davon ausgegangen werden, dass er zum selben Bereich gehört. Andererseits kann beispielsweise ein näher am Saatpunkt liegender Bildpunkt höher gewichtet werden als ein weiter entfernt liegender, da unter der Annahme, dass der Saatpunkt recht zentral in dem gesuchten Bereich liegt, näher daran liegende Punkte ebenfalls noch im gesuchten Bereich liegen. Gibt man diesen Punkten ein höheres Gewicht für die statistische Auswertung, insbesondere für die Mittelwertbildung bezüglich des Referenzwerts, kann eine genauere Bestimmung des zu segmentierenden Bereichs erfolgen.

Nach einer weiteren vorteilhaften Weiterbildung der Erfindung kann die Segmentierungsvorrichtung eine Bereichskanten-Bestimmungseinrichtung zur Bestimmung von Bereichskanten anhand eines Sprungs in der verglichenen Eigenschaft der Bildpunkte besitzen. Eine solche Bereichskanten-Bestimmungseinrichtung nutzt die Überlegung, dass zu segmentierende Zähne und/oder Gebissabschnitte durch an sich glatte Begrenzungskanten begrenzt werden, und zwar sowohl zum Zahnfleisch hin als auch zu einem jeweils benachbarten Zahn sowie darüber hinaus auch am freien Ende des Zahns, so dass anders als bei ausgefransten Konturen eine glatte Konturlinie als Begrenzung bestimmt werden muss. Bestimmt die Segmentierungsvorrichtung für einen Bildpunkt eine sprunghafte Veränderung des Homogenitätskriteriums im Vergleich zu benachbarten Bildpunkten, kann mit großer Wahrscheinlichkeit darauf geschlossen werden, dass ein solcher Bildpunkt ein Begrenzungspunkt der Kontur ist. Beispielsweise wird am Zahnfleischrand eine sprunghafte Änderung des Farbraums eintreten, da von der üblicherweise weißen Farbe des Zahns auf die rosa Farbe des Zahnfleischs übergegangen wird. Werden auf diese Weise zumindest einige Bildpunkte als Begrenzungspunkte identifiziert, kann die Zahnkontur sauber segmentiert werden, indem derartige Begrenzungspunkte miteinander durch eine gerade und/oder kontinuierliche Kurve verbunden werden. Mittels einer solchen Bestimmung von Bereichskanten können an einzelnen Stellen nicht sauber identifizierbare Konturränder und Ausreißer eliminiert werden und die Zahnkontur sauber festgelegt werden.

Alternativ oder zusätzlich kann in Weiterbildung der Erfindung eine Abgleichseinrichtung vorgesehen sein, mittels derer der jeweils segmentierte Zahn und/oder der jeweils segmentierte Gebissabschnitt mit einem gespeicherten Sollbild des zu segmentierenden Zahns und/oder Gebissbilds abgeglichen wird. Hierzu kann insbesondere ein statistisches Abweichungsmodell Verwendung finden, mittels dessen Abweichungen des segmentierten Zahns und/oder Gebissabschnitts von einem Mittelwert der gespeicherten Sollbilder bestimmt wird und/oder ein zulässiger Abweichungskorridor von diesem Mittelwert der gespeicherten Sollbilder vorgegeben wird. Insbesondere kann mittels eines solchen statistischen Modells die zu segmentierende Zahnkontur in die Sollkontur des jeweiligen Zahns bzw. Gebissabschnitts hineinwachsen, wodurch eine sauberere, weniger fehlerbehaftete Bereichsbestimmung erreicht wird.

Um insbesondere bei schwieriger zu erkennenden Bereichskanten dennoch eine sichere Bestimmung der Bereichsgrenzen zu erzielen, kann in Weiterbildung der Erfindung auch mit einer Annäherung von beiden Seiten her gearbeitet werden, d.h. es wird nicht nur der an sich auszuschneidende Bereich segmentiert, sondern auch der diesen Bereich umgebende, sozusagen negative Bereich. Insbesondere kann mit einem Bereichswachstumsverfahren von zwei Saatpunkten aus, von denen einer im zu segmentierenden Bereich liegt und einer außerhalb des zu segmentierenden Bereichs liegt, sozusagen aufeinander zu gearbeitet werden. Soll beispielsweise die obere Zahnreihe segmentiert werden, kann ein Saatpunkt in der Zahnreihe und ein Saatpunkt im Zahnfleisch gesetzt werden, so dass der Bereich der zu segmentierenden Zähne auf den Zahnfleischrand zuwächst und der diesen umgebende Zahnfleischbereich von oben her auf die Zahnfleisch-/Zahngrenze zuwächst. Je nachdem, welche Zahnbehandlungsfunktion durchzuführen ist, kann die Bildmanipulationsvorrichtung verschiedene Werkzeuge aufweisen. In Weiterbildung der Erfindung kann die Bildmanipulationsvorrichtung insbesondere eine Zahnersatz-Bestimmungseinrichtung zur automatischen Bestimmung eines Ersatzzahnes in Abhängigkeit des segmentierten Bildausschnitts aufweisen. Soll beispielsweise ein einzelner Zahn ersetzt werden, bestimmt die Zahnersatzbestimmungseinrichtung automatisch anhand des segmentierten Bildausschnitts den am besten passenden Ersatzzahn, der hinsichtlich Kontur und/oder Farbe bestmöglich passt.

Um aus einer Vielzahl von gespeicherten Ersatzzahnmodellen den bestpassenden richtig auszuwählen, kann die Zahnersatzbestimmungseinrichtung eine Normierungseinrichtung aufweisen, mittels derer der segmentierte Zahn bzw. Gebissabschnitt zunächst auf eine Sollhöhe normiert wird, und zwar insbesondere auf einen Mittelwert der Höhenwerte aller Zähne eines gespeicherten Referenzmodells. Auf Basis dieser normierten Zahnhöhe wird dann mittels einer Konturvergleichsvorrichtung ein nächstkommender Referenzzahn bzw. ein nächstkommender Referenzgebissabschnitt aus einer Vielzahl von gespeicherten Referenzzähnen und/oder Referenzgebissabschnitten ausgewählt.

Vorteilhafterweise werden hierbei gespeicherte zweidimensionale Darstellungen von Referenzzähnen bzw. Referenzgebissabschnitten verwendet. Alternativ oder zusätzlich kann jedoch auch vorgesehen sein, dass die Zahnersatzbestimmungseinrichtung bzw. die Konturvergleichsvorrichtung Bilder bzw. Darstellungen aus 3D-Datenbanken verwendet und diese dreidimensionalen Bilder entsprechend transformiert, d.h. in eine zweidimensionale Bilddarstellung transformiert und mit der richtigen Ausrichtung des Zahns bzw. Gebissabschnittes in das System als 2D-Bild einfügt. Hierdurch kann eine Schnittstelle zu dreidimensional arbeitenden Systemen, 3D-Datenbanken und CAD-Systemen geschaffen werden. Im Sinne einer einfachen Ausbildung mit geringen Datenmengen und Rechenschritten ist vorteilhafterweise jedoch die Verwendung von zweidimensionalen Referenzdarstellungen vorgesehen.

Ist der jeweilige Ersatzzahn bzw. Gebissabschnitt ausgewählt, wird dessen Kontur automatisch an den segmentierten Bildausschnitt angepasst. Dies kann insbesondere durch Skalierung mit dem zuvor bestimmten Normierungsfaktor, mit dem der segmentierte Bildausschnitt auf den Mittelwert der Zahnsollhöhe normiert wurde, erfolgen. Alternativ oder zusätzlich kann auch die seitliche Kontur an die Kontur der Zahnlücke, in die der Ersatzzahn einzusetzen ist, angepasst werden. Bei der Ersatzzahnbehandlung und deren Visualisierung ist es insofern vorteilhaft, nicht nur den zu ersetzenden Zahn zu segmentieren, sondern auch die Zahnlücke zu segmentieren, insbesondere dadurch, dass die seitlichen Zähne, die die Zahnlücke begrenzen, segmentiert werden. Hierdurch können eventuell vorhandene Zahnlücken zwischen dem zu ersetzenden Zahn im Originalgebiss und den angrenzenden Zähnen berücksichtigt werden.

Die Konturanpassung kann vorteilhafterweise mittels eines vorab gespeicherten Statistikmodells erfolgen, das gespeicherte Konturmittelwerte als Sollkontur vorgibt. Alternativ dazu kann auch vorgesehen werden, dass der Ersatzzahn/ die Ersatzzähne aus dem Ist-Bild durch Verwendung des/der kontralateralen Zahnes/zähne durch Spiegelung abgeleitet wird.

Um die Visualisierung von Schwankungen bei der Bildgenerierung unabhängig zu machen und/oder unterschiedliche Belichtungsverhältnisse zu eliminieren, ist in Weiterbildung der Erfindung eine Kalibriervorrichtung vorgesehen, mittels derer die Ist-Darstellung des Gebisses hinsichtlich Größe und/oder Farbe und/oder Grauton kalibriert wird. Dies kann insbesondere dadurch erfolgen, dass auf dem zu erzeugenden Ist-Bild des zu behandelnden Gebisses Referenzobjekte mit abgebildet werden, deren Größe und/oder Farbton und/oder Helligkeit und/oder Sättigung und/oder Grauton bekannt ist. Insbesondere kann ein Sortiment von Referenzzähnen und/oder eine Längenskala wie beispielsweise ein Lineal mit abgebildet werden, so dass einerseits der zu segmentierende Zahn bzw. Gebissabschnitt hinsichtlich Farbton durch Zuordnung zum nächstkommenden Referenzmodell sowie andererseits hinsichtlich seiner Abmessungen wie Zahnhöhe und Zahnbreite bestimmbar ist.

Die Erfindung wird nachfolgend anhand eines bevorzugten Ausführungsbeispiels und zugehöriger Zeichnungen näher erläutert. In den Zeichnungen zeigen:
- Fig. 1:: eine schematische Seitenansicht der Ist-Bilderzeugungsvorrichtung mit der Kopfpositioniervorrichtung zur Positionierung des Kopfes eines Patienten sowie der relativ hierzu montierten Kamera,
- Fig. 2:: eine schematische Frontansicht des von der Kopfpositioniervorrichtung positionierten Patientenkopfes sowie der bei der Erzeugung des Ist-Bilds in das Bild platzierten Kalibriervorrichtung,
- Fig. 3:: die Gebissbild-Simulationsvorrichtung mit einer Ist-Darstellung des Gebisses in seinem Ist-Zustand, und
- Fig. 4:: eine schematische Seitenansicht der Ist-Bilderzeugungsvorrichtung mit einem Patienten in Seitenansicht sowie der relativ hierzu montierten Kamera zur Erzeugung eines Ist-Bilds des Patientenkopfs, um zusammen mit der Frontansicht eine 3-D Simulation des Gebissbilds zu erzeugen.

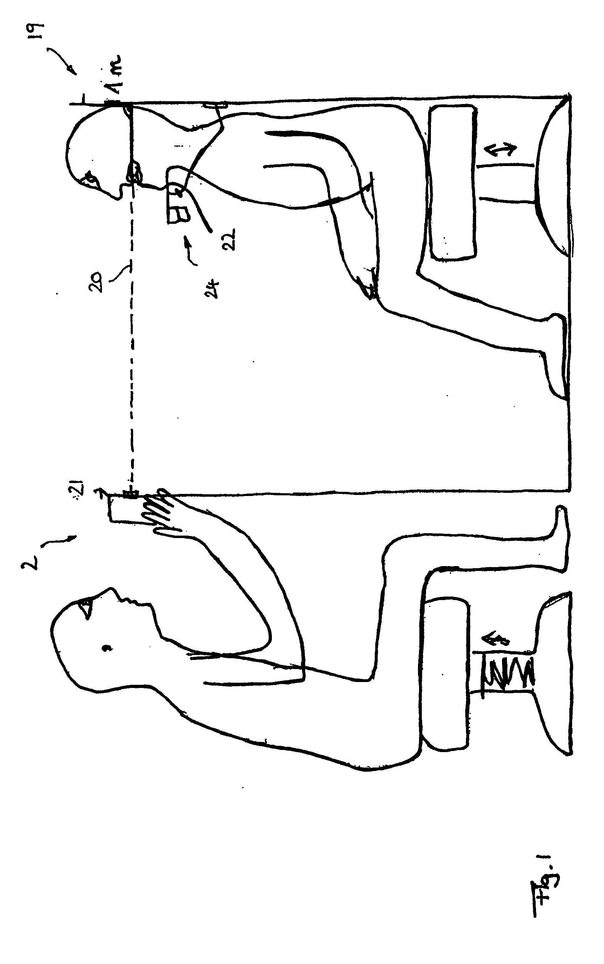

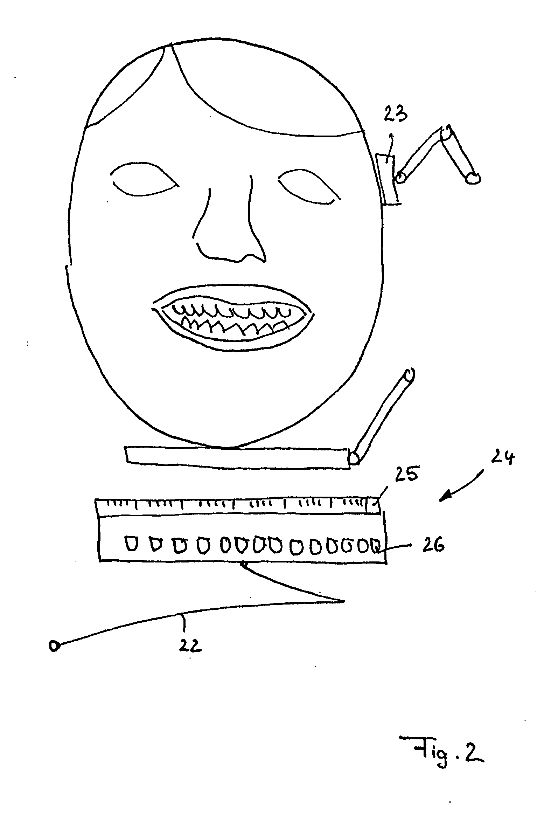

Die in den Zeichnungen dargestellte Gebissbild-Simulationsvorrichtung 1 umfasst die in Fig. 1 gezeigte Kopfpositioniervorrichtung 19, zu der relativ in vorbestimmter Position eine Kamera 21 montiert ist. Die Kopfpositioniervorrichtung 19 umfasst in der gezeichneten Ausführung vorteilhafterweise eine höhen- und winkeleinstellbare Kinnstütze 22, zumindest eine einstellbare Seitenstütze 23 sowie eine ebenfalls einstellbare Hinterkopfstütze 24, vgl. Figuren 1 und 2. Mittels dieser einstellbaren Stützen kann die Kopfpositioniervorrichtung 19 den Kopf des Patienten derart ausrichten, dass die Okklusionsebene 20, die von den Zahnkauflächen bei geschlossenem Gebiss definiert wird, exakt ausgerichtet werden kann, und zwar derart, dass das Objektiv der Kamera 21 in der genannten Okklusionsebene 20 zu liegen kommt und die Bildhorizontale in der genannten Okklusionsebene 20 liegt. Durch ein derartiges Ausrichten der Kopfposition kann erzielt werden, dass das Gebiss in einer exakt identischen Position abgebildet wird, und zwar insbesondere in der Position, in der auch die Referenzzähne der gespeicherten Referenzzahnmodelle abgebildet sind.

Wie Fig. 2 zeigt, wird bei der Generierung der Ist-Darstellung eine Kalibriervorrichtung 24 umfassend einen Längenmaßstab 25 in Form eines Lineals sowie einen Satz von Referenzzähnen 26 in das Bild eingeblendet. Die genannte Kalibriervorrichtung 24 kann hier beispielsweise an einem abwinkelbaren Haltearm 27 befestigt sein, mittels derer der Längenmaßstab 25 sowie die Referenzzähne 26 unter der Kinnstütze 22 positioniert werden können.

Um den Kopf exakt in der gewünschten Position ausrichten zu können, kann die Kopfpositioniervorrichtung 19 vorteilhafterweise weitere Hilfsmittel aufweisen, wie beispielsweise ein Beißspatel, auf das der Patient zu beißen hat und mittels dessen die Position des Kopfes exakt in der genannten Weise ausgerichtet werden kann, während die diversen einstellbaren Stützen eingestellt werden. Die variable Einstellbarkeit hat den Hintergrund, dass das Gebiss nicht immer in exakt derselben Ausrichtung innerhalb der Kopfkontur positioniert ist.

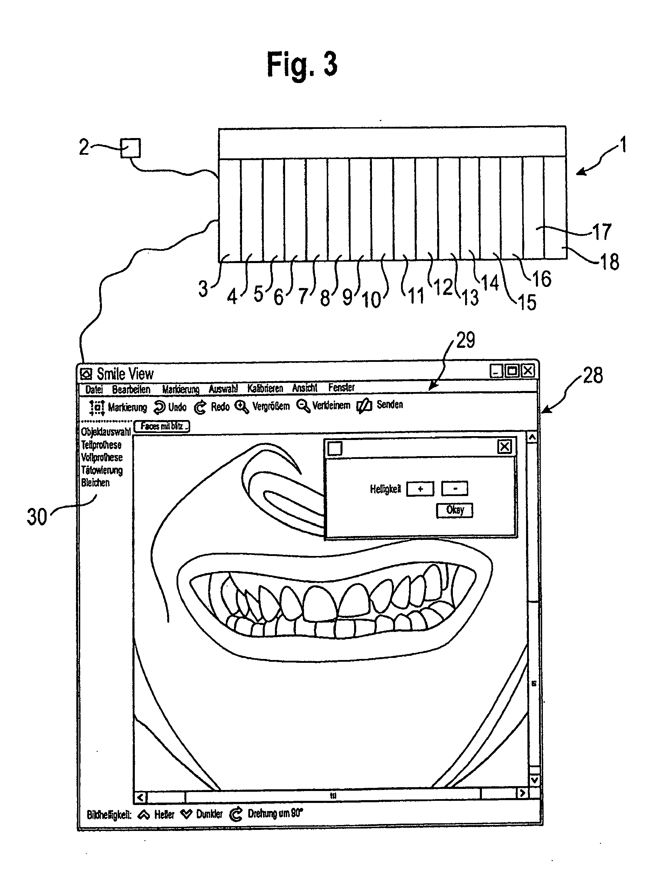

Mittels der Kamera 21 wird sodann ein digitales Bild des Gebisses erzeugt, das auf einem Bildschirm 28 der Gebissbildsimulationsvorrichtung 21 digital angezeigt werden kann, vgl. Fig. 3. Die Ist-Darstellung ist hierbei in einen Menürahmen 29 eingeblendet, über den diverse Steuerbefehle eingegeben werden können. Wie Fig. 3 zeigt, kann beispielsweise durch Anklicken der linken Menüleiste 30 die gewünschte Zahnbehandlungsfunktion ausgewählt werden, wie beispielsweise eine Zahnersatzfunktion durch Anklicken des Befehls "Teilprothese" bzw. "Vollprothese".

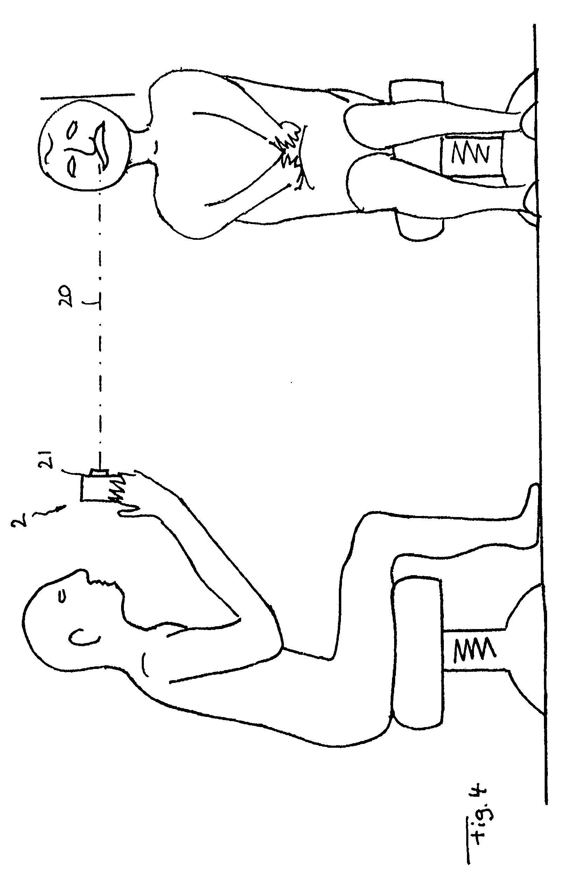

Um eine einfache und schnelle Datenverarbeitung zu erreichen, kann ein zweidimensionales Gebissbild erzeugt bzw. simuliert werden. Für diesen Fall ist die Erzeugung eines Ist-Bilds des Patienten in Frontansicht gemäß Figur 1 ausreichend. Soll jedoch eine dreidimensionale Gebissbildsimulation erzeugt werden, wird vorteilhafterweise zumindest eine zweite Ist-Darstellung des Gebisses bzw. des Patientenkopfs erzeugt, und zwar vorteilhafterweise eine Seitenansicht, wie dies Figur 4 zeigt. Auch hier wird der Kopf mittels der Kopfpositioniereinrichtung 19 in die entsprechend ausgerichtete Position gebracht, so dass die Linse der Kamera in der Okklusionsebene liegt. Aus den beiden 2-D-Bildern kann dann die gewünschte 3-D-Darstellung errechnet werden.

Soll beispielsweise die gesamte obere Zahnreihe ersetzt werden, ist in der Ist-Darstellung der die obere Zahnreihe wiedergebende Bildabschnitt auszuschneiden. Hierzu umfasst die Gebissbild-Simulationsvorrichtung 1 eine Segmentierungsvorrichtung 5, die die obere Zahnreihe segmentiert. Die Segmentierungsvorrichtung 5 kann hierbei nach einer Ausführung der Erfindung eine Bereichswachstums-Bestimmungseinrichtung 8 aufweisen, die von einem Saatpunkt ausgehend in der eingangs beschriebenen Weise einen entsprechenden Bildbereich anhand eines Homogenitätskriteriums oder mehrerer solcher Homogenitätskriterien, wie zuvor beschrieben, identifiziert. Der Saatpunkt kann hierbei grundsätzlich manuell gesetzt werden, beispielsweise durch Anklicken eines der Zähne durch Positionieren eines Cursors und Anklicken des entsprechenden Punkts. Alternativ kann der Saatpunkt auch automatisch gesetzt werden, wozu die Segmentierungsvorrichtung 5 einen Saatpunktgenerator 7 besitzen kann, der beispielsweise einen Zahn anhand der Bildhelligkeit und auch der vorbekannten Information identifizieren kann, dass die Kauebene bzw. Okklusionsebene in der Ebene des Kameraobjektivs und damit in einem bestimmten Bildabschnitt liegt.

Die Bereichswachstums-Bestimmungseinrichtung 8 analysiert hierbei von dem Saatpunkt ausgehend Pixel für Pixel das Bild mit einem Bildpunktanalysator 9, der Bildpunkteigenschaften wie beispielsweise den Farbraumvektor eines Bildpunkts analysiert und mittels einer Vergleichseinrichtung 10 mit einem entsprechenden Referenzwert vergleicht. Der Referenzwert kann durch einen Vergleichswertgenerator 11 adaptiv bestimmt werden. Insbesondere kann wie eingangs erläutert durch einen Mittelwertbildner 12 der Vergleichswert anhand der Mittelwerte zuvor bestimmter Bildpunkteigenschaften angepasst werden.

Ist von der Segmentiervorrichtung 5 die Darstellung der oberen Zahnreihe segmentiert, wird diese zunächst mittels einer Normierungseinrichtung 16 auf eine normierte Zahnhöhe normiert, woraufhin eine Konturvergleichsvorrichtung 17 aus einer Datenbank, in der diverse Zahn- und Gebissmodelle abgespeichert sind, das am nächstkommende Gebissmodell für die zu ersetzende obere Zahnreihe ausgewählt wird. Wird nur die obere Zahnreihe ersetzt, wird dabei das entsprechende Referenzzahnmodell ausgewählt, das hinsichtlich Farbe der unteren Zahnreihe am nächsten kommt. Alternativ oder zusätzlich kann das ausgewählte Referenzzahnmodell hinsichtlich seines Farbtons entsprechend angepasst werden.

Das ausgewählte Referenzzahnmodell wird ebenso hinsichtlich seiner Form und Größe an die Kontur des segmentierten Gebissabschnitts angepasst. Eine Konturanpassungsvorrichtung 18 streckt bzw. staucht die Darstellung des ausgewählten Referenzzahnmodells derart, dass es sich bestmöglich in den segmentierten Bildabschnitt einfügt.

## Patentansprüche

1. Gebissbild-Simulationsvorrichtung zur Simulation eines Gebissbilds zur Behandlungsergebnis-Visualisierung und/oder zur Generierung von Daten für die Herstellung von Zahnersatzteilen, mit einer Ist-Bilderzeugungsvorrichtung (2) zur Erzeugung einer Ist-Darstellung des Gebisses in seinem Ist-Zustand, einer Bild-Manipulationsvorrichtung (3) zur Manipulation von Bilddaten der Ist-Darstellung anhand einer vorgebbaren Gebiss- und/oder Zahnbehandlungsfunktion, und einer Soll-Bilderzeugungsvorrichtung (4) zur Erzeugung einer Soll-Darstellung des Gebisses in seinem Soll-Zustand anhand der Ist-Darstellung und der manipulierten Bilddaten, **dadurch gekennzeichnet, dass** die Bild-Manipulationsvorrichtung (3) eine Segmentierungsvorrichtung (5) zur automatischen Segmentierung eines Zahns und/oder eines Gebissteils in der Ist-Darstellung in Abhängigkeit der vorgegebenen Gebiß- und/oder Zahnbehandlungsfunktion sowie einen Segmentmanipulator (6) zur automatischen Manipulation des segmentierten Bildausschnitts in Abhängigkeit der vorgegebenen Gebiß- und/oder Zahnbehandlungsfunktion aufweist.

2. Gebissbild-Simulationsvorrichtung nach dem vorhergehenden Anspruch, wobei die Ist-Bilderzeugungsvorrichtung (2) eine Kopfpositioniervorrichtung (19) zur Positionierung des Kopfes einer Person, deren Gebissbild zu simulieren ist, in einer vorgegebenen Ausrichtung und einem vorgegebenen Abstand relativ zu einer Kamera derart, dass die Linse der Kamera in der von den Kauflächen der Zähne des geschlossenen Gebisses definierten Kauebene liegt.

3. Gebissbild-Simulationsvorrichtung nach dem vorhergehenden Anspruch, wobei die Kopfpositioniervorrichtung (19) eine einstellbare Kinnstützfläche sowie zumindest eine weitere Kopfstützfläche aufweist.

4. Gebissbild-Simulationsvorrichtung nach einem der beiden vorhergehenden Ansprüche, wobei die Kopfpositioniervorrichtung (19) eine Ausrichthilfe mit einer Beißfläche zur Ausrichtung des Gebisses relativ zur Kamera (21) aufweist.

5. Gebissbild-Simulationsvorrichtung nach einem der vorhergehenden Ansprüche, wobei die Bild-Manipulationsvorrichtung (3) eine Kalibriervorrichtung zur Kalibrierung der Ist-Darstellung des Gebisses hinsichtlich Größe und/oder Farbe und/oder Grauton aufweist, wobei die genannte Kalibriervorrichtung (24) Referenzzähne (26) und/oder einen Längenmaßstab (25) umfasst, die mittels eines Haltearms (27) unter der Kinnstütze (22) der Kopfpositioniervorrichtung (19) positionierbar sind.

6. Gebissbild-Simulationsvorrichtung nach einem der vorhergehenden Ansprüche, wobei die Bild-Manipulationsvorrichtung (3) eine Zahnersatz-Bestimmungseinrichtung (15) zur Bestimmung eines Ersatzzahnes in Abhängigkeit des segmentierten Bildausschnitts aufweist, wobei die Zahnersatz-Bestimmungseinrichtung (15) eine Normierungseinrichtung (16) zur Normierung des segmentierten Zahns und/oder Gebissabschnitts auf eine Soll-Höhe, insbesondere auf einen Mittelwert der Höhenwerte aller Zähne eines gespeicherten Referenzmodells, aufweist, und eine KonturvergleichsVorrichtung (17) zur Bestimmung eines nächstkommenden Referenzzahnes und/oder Referenz-Gebissabschnitts aus einer Vielzahl von gespeicherten Referenzzähnen und/oder Referenz-Gebissabschnitten aufweist.

7. Gebissbild-Simulationsvorrichtung nach dem vorhergehenden Anspruch, wobei die gespeicherten und/oder zu verwendenden Referenzzähne und/oder gespeicherten und/oder zu verwendenden Referenz-Gebissabschnitte Zahn- und/oder Gebissdarstellungen umfassen, die den jeweiligen Zahn- und/oder Gebissabschnitt in derselben Ausrichtung und in demselben Abstand zeigen, in der und in dem der Zahn- und/oder Gebissabschnitt in der Ist-Darstellung der Ist-Bilderzeugungsvorrichtung (2) gezeigt ist, wobei die Zahnersatz-Bestimmungseinrichtung (15) eine Kontur-Anpassungsvorrichtung (18) zur automatischen Anpassung der Kontur des ausgewählten Referenzzahnes und/oder Referenz-Gebissabschnitts an den segmentierten Bildausschnitt aufweist, wobei die Kontur-Anpassungsvorrichtung (18) ein vorab gespeichertes Statistikmodel zur Anpassung der Zahnkontur anhand gespeicherter Konturmittelwerte umfasst.

8. Gebissbild-Simulationsvorrichtung nach dem vorhergehenden Anspruch, wobei die Segmentierungseinrichtung (5) einen Saatpunktgenerator (7) zur Erzeugung eines Saatpunkts innerhalb und/oder außerhalb des zu segmentierenden Zahns und/oder Gebissabschnitts sowie eine Bereichswachstums-Bestimmungseinrichtung (8) zur Bestimmung eines vom Saatpunkt aus wachsenden Bildbereichs anhand zumindest eines Homogenitätskriteriums für einzelne Bildpunkte des Bildbereichs aufweist, wobei die Bereichswachstums-Bestimmungseinrichtung (8) einen Bildpunktanalysator (9) zur Bestimmung zumindest einer Eigenschaft eines dem Saatpunkt oder einem zuvor analysierten Bildpunkt benachbarten Bildpunkts, eine Vergleichseinrichtung (10) zum Vergleichen der bestimmten Eigenschaft eines jeweiligen Bildpunkts mit einem Wert dieser Eigenschaft für den Saatpunkt und/oder den zuvor analysierten Bildpunkt sowie zum Zuweisen des Bildpunkts zum Bildbereich in Abhängigkeit des Vergleichs, insbesondere in Abhängigkeit des Abgleichs der Abweichung mit einem Schwellwert, aufweist.

9. Gebissbild-Simulationsvorrichtung nach dem vorhergehenden Anspruch, wobei die zumindest eine Eigenschaft einen Grauwert, einen Farbton, eine Sättigung und/oder eine Helligkeit des Bildpunkts umfasst, und/oder als Homogenitätskriterium ein die Primärfarben Rot, Grün und Blau angebender Farbvektor und/oder ein den Farbton, die Sättigung und die Helligkeit angebender Farbvektor eines jeden Bildpunkts vorgesehen ist.

10. Gebissbild-Simulationsvorrichtung nach einem der beiden vorhergehenden Ansprüche, wobei die Vergleichseinrichtung (10) einen adaptiven Vergleichswertgenerator (11) zur adaptiven Bestimmung eines Vergleichswerts für die zu vergleichende Eigenschaft anhand der zuvor bestimmten Eigenschaftswerte zuvor untersuchter Bildpunkte aufweist, wobei der adaptive Vergleichswertgenerator (11) insbesondere einen Mittelwertbildner (12) zur Bildung eines Mittelwerts der zuvor bestimmten Eigenschaftswerte zuvor untersuchter Bildpunkte sowie einen Gewichtungsgeber zur Gewichtung einzelner Bildpunkte anhand ihrer Abweichung von einem zuvor bestimmten Mittelwert aufweist.

11. Gebissbild-Simulationsvorrichtung nach einem der vorhergehenden Ansprüche, wobei die Segmentierungseinrichtung (5) eine Bereichskanten-Bestimmungseinrichtung (13) zur Bestimmung von Bereichskanten anhand eines Sprungs in der verglichenen Eigenschaft der Bildpunkte und zur Verbindung solchermaßen identifizierter Bildpunkte aufweist.

12. Gebissbild-Simulationsvorrichtung nach einem der vorhergehenden Ansprüche, wobei die Segmentierungseinrichtung (5) eine aktive Konturbestimmungseinrichtung zur Bestimmung der Kontur eines zu segmentierenden Zahn- und/oder Gebißteils mittels einer parametrisierten Kurve aufweist, wobei die aktive Konturbestimmungseinrichtung eine Gradientenvektor-Bestimmungseinrichtung zur Bestimmung von Gradientenvektoren anhand von Bildpunkteigenschaften eines von der parametrisierten Kurve umschlossenen Bildbereichs und/oder eines die parametrisierte Kurve umgebenden Bildbereichs, und eine Konturformungseinrichtung zur Verformung der parametrisierten Kurve in Abhängigkeit der bestimmten Gradientenvektoren aufweist.

13. Gebissbild-Simulationsvorrichtung nach einem der vorhergehenden Ansprüche, wobei die Segmentierungseinrichtung (5) eine Abgleichseinrichtung (14) zum Abgleich des segmentierten Zahns und/oder Gebissabschnitts mit einem gespeicherten Soll-Bild des zu segmentierenden Zahns und/oder Gebissbilds aufweist, wobei die Abgleichseinrichtung (14) ein statistisches Abweichungsmodel zur Bewertung von Abweichungen des segmentierten Zahns und/oder Gebissabschnitts von einem Mittelwert der gespeicherten Soll-Bilder und/oder zur Vorgabe eines zulässigen Abweichungskorridors aufweist.

14. Gebissbild-Simulationsvorrichtung nach Anspruch 8 oder einem der darauf rückbezogenen Ansprüche, wobei die Bereichswachstums-Bestimmungseinrichtung (8) von zwei Saatpunkten ausgehend, von denen einer im zu segmentierenden Bereich und einer im nicht zu segmentierenden Bereich generiert ist, zwei aufeinander zuwachsende Bildbereiche und deren Trennlinie voneinander bestimmt.

15. Gebissbild-Simulationsvorrichtung nach einem der vorhergehenden Ansprüche, wobei die Bild-Manipulationsvorrichtung (3) den Segmentmanipulator beinhaltet, der nach erfolgter Auswahl der Behandlungsfunktion und Segmentierung die Manipulation der Ist-Darstellung des Gebisses hinsichtlich Größe, Form, Farbe, Sättigung, Ausrichtung und/oder Position ermöglicht.
